Europäisches Patentamt

European Patent Office

Office européen des brevets

(11) Veröffentlichungsnummer: **0 043 924**
**B1**

# (12) EUROPÄISCHE PATENTSCHRIFT

(45) Veröffentlichungstag der Patentschrift:
04.05.83

(51) Int. Cl.³: **C 07 D 201/16**

(21) Anmeldenummer: **81104595.4**

(22) Anmeldetag: **15.06.81**

(54) **Verfahren zur kontinuierlichen Extraktion von Caprolactam aus solches und dessen Oligomere enthaltenden wässrigen Lösungen.**

(30) Priorität. 12.07.80 DE 3026538

(43) Veröffentlichungstag der Anmeldung:
20.01.82 Patentblatt 82/3

(45) Bekanntmachung des Hinweises auf die Patenterteilung:
04.05.83 Patentblatt 83/18

(84) Benannte Vertragsstaaten:
BE CH DE FR GB IT LI NL

(56) Entgegenhaltungen:
CH-A-417 600
DE-A-2 656 182
DE-A-2 724 361
DE-B-2 550 934

(73) Patentinhaber: **BASF Aktiengesellschaft,
Carl-Bosch-Strasse 38, D-6700 Ludwigshafen (DE)**

(72) Erfinder: **Fuchs, Hugo, Dr., Egellstrasse 28,
D-6700 Ludwigshafen (DE)**
Erfinder: **Brand, Uwe, Rheingoldstrasse 12,
D-6841 Rosengarten (DE)**
Erfinder: **Deuker, Ernst, Dr., Westring 31,
D-6718 Gruenstadt (DE)**
Erfinder: **Frommer, Elmar, Dr., Lisztstrasse 117,
D-6700 Ludwigshafen (DE)**

## Verfahren zur kontinuierlichen Extraktion von Caprolactam aus solches und dessen Oligomere enthaltenden wäßrigen Lösungen

Bei der Extraktion von Polycaprolactam mit Wasser zur Entfernung von Monomeren erhält man wäßrige Lösungen, die Caprolactam und dessen Oligomere enthalten. Es ist erforderlich, monomeres Caprolactam aus solches enthaltenden Lösungen wiederzugewinnen.

Aus der DD-PS 9 215 ist bekannt, Caprolactam enthaltende Waschwässer zunächst teilweise einzuengen, dabei ausgefallene Oligomere durch Filtration zu entfernen, das Filtrat mit Lösungsmitteln zu extrahieren und aus der Lösungsmittelphase das Caprolactam zu isolieren. Eine solche Arbeitsweise ist jedoch energetisch aufwendig, und außerdem bereitet die Filtration des ausgefallenen Oligomeren Schwierigkeiten. Entsprechend der DE-OS 1 770 088 führt man die Extraktion von Caprolactam enthaltenden Waschwässern mit einem mit Wasser nicht mischbaren Lösungsmittel durch, wobei das Lösungsmittel einen Verteilungskoeffizienten bei 25°C für Caprolactam im System Lösungsmittel/Wasser von mindestens 0,2 hat und eine kleinere Dichte als die wäßrige Phase oder eine größere Dichte als die der oligomeren Feststoffe aufweist. Bei der technischen Verwirklichung eines solchen Verfahrens im kontinuierlichen Betrieb treten jedoch immer wieder Schwierigkeiten durch oligomere Feststoffe auf, insbesondere treten Verstopfungen der Extraktionskolonnen ein.

Es war deshalb die technische Aufgabe gestellt, die Extraktion von Caprolactam aus solches und dessen Oligomere enthaltenden wäßrigen Lösungen so zu gestalten, daß die Extraktion reibungslos verläuft und keine Störungen durch die Oligomeren auftreten.

Diese Aufgabe wird gelöst in einem Verfahren zur kontinuierlichen Extraktion von Caprolactam aus solches und dessen Oligomere enthaltenden wäßrigen Lösungen mit organischen Lösungsmitteln, wobei man im oberen Teil einer Extraktionszone eine Caprolactam und dessen Oligomere enthaltende wäßrige Lösung und im unteren Teil organische Lösungsmittel zuführt, am oberen Ende eine Lösung von Caprolactam im organischen Lösungsmittel und im unteren Ende eine Oligomeren enthaltende wäßrige Phase abführt, dadurch gekennzeichnet, daß man flüssige aromatische Kohlenwasserstoffe verwendet und die Extraktion bei einem pH-Wert von 2,0 bis 6,0 durchführt.

Das neue Verfahren hat den Vorteil, daß die Extraktion reibungslos verläuft und keine Störungen durch die Oligomeren auftreten.

Die zur extrahierenden wäßrigen Lösungen enthalten in der Regel 2 bis 20 Gew.-% Caprolactam und bezogen auf die in der wäßrigen Lösung enthaltenen organischen Verbindungen 15 bis 25 Gew.-% Oligomere des Caprolactams. Besonders bevorzugte Lösungen enthalten 3 bis 10 Gew.-% Caprolactam und die entsprechenden Mengen an Oligomeren. Solche Lösungen erhält man z. B. durch Extraktion von Polycaprolactam mit Wasser, wie sie beispielsweise in DE-PS 2 242 641 beschrieben wird. Zusätzlich können bei der Extraktion noch Caprolactam enthaltende wäßrige Lösungen anderen Ursprungs mitverwendet werden, z. B. wäßrige Lösungen, die bei der Extraktion von Rohlactam anfallen.

Die Extraktion führt man mit flüssigen aromatischen Kohlenwasserstoffen, wie Benzol, Xylol oder Toluol durch. Bevorzugte aromatische Kohlenwasserstoffe sind Benzol oder Toluol. Vorteilhaft wendet man je Gewichtsteil wäßrige Lösung die 2- bis 12fache, insbesondere 2,5- bis 10fache Gewichtsmenge an flüssigen aromatischen Kohlenwasserstoffen an. Die Extraktion führt man vorzugsweise bei einer Temperatur von 30 bis 70°C und unter Atmosphärendruck oder schwach erhöhtem Druck z. B. bis zu 1,5 bar durch.

Erfindungsgemäß führt man die Extraktion bei einem pH-Wert von 2,0 bis 6,0, insbesondere 2,5 bis 5,0 durch. Verwendet man Caprolactam und dessen Oligomere enthaltende Waschwässer allein, so säuert man diese zweckmäßig vor der Extraktion mit nichtoxidierenden Mineralsäuren, wie Schwefelsäure oder Phosphorsäure, insbesondere Schwefelsäure bis zu dem obengenannten pH-Wert an. Werden zusätzlich Caprolactam enthaltende wäßrige Lösungen, die bei der Extraktion von Rohlactam, die z. B. nach der DE-PS 1 194 863 anfallen, mitverwendet, so erübrigt sich ein Ansäuern, da diese wäßrigen Lösungen bereits von sich aus den gewünschten pH-Wert aufweisen.

Man verfährt vorteilhaft so, daß man in der Extraktionszone im oberen Fünftel die Caprolactam und dessen Oligomere enthaltende wäßrige Lösung und im unteren Fünftel flüssige aromatische Kohlenwasserstoffe zugibt, am oberen Ende der Kolonne eine Lösung von Caprolactam in flüssigen aromatischen Kohlenwasserstoffen und im unteren Ende eine Oligomeren des Caprolactams enthaltende wäßrige Phase entnimmt. Vorteilhaft wird die Extraktion in üblichen Extraktionskolonnen, z. B. Siebbodenkolonnen, Füllkörperkolonnen, pulsierten Füllkörpern oder Rührscheibenkolonnen durchgeführt. Geeignete Kolonnen haben beispielsweise 10 bis 50 Böden.

Die erhaltene Lösung von Caprolactam in flüssigen aromatischen Kohlenwasserstoffen, die z. B. 0,5 bis 8 Gew.-% Caprolactam enthält, wird vorteilhaft als Extraktionsmittel bei der Extraktion von Rohlactam, wie sie in der DE-PS 2 656 182 beschrieben wird, verwendet. Aus der wäßrigen Phase können die Oligomeren des Caprolactams abgetrennt werden und z. B. durch thermische Spaltung auf monomeres Caprolactam aufgearbeitet werden.

Das Verfahren nach der Erfindung sei an folgendem Beispiel veranschaulicht.

## Beispiel

Bei der Herstellung von Caprolactam durch Beckmann'sche Umlagerung fällt ein Rohlactam an, das durch Extraktion mit Benzol gereinigt wird. Die anfallende Benzol-Lactam-Phase wird zu Caprolactam aufgearbeitet. Als wäßrige Phase fällt eine braune Lösung an, die Ammonsulfat, Verunreinigungen und wenig Caprolactam enthält.

10 t dieser braunen wäßrigen Phase mit einem Lactamgehalt von 1,0 Gew.-% und einem pH-Wert von 4,2 werden stündlich dem obersten von 25 Böden einer Siebbodenkolonne mit einem Durchmesser von 2500 mm zugeführt. Dem untersten Boden werden stündlich 50 549 kg/h Benzol zugeführt. Die Temperatur in der Extraktionskolonne beträgt 60 bis 65°C. Zusätzlich werden stündlich 10 m³ eines Extraktionswassers aus der Polyamidherstellung mit 4% Caprolactam und 0,8% Oligomeren mit einem pH-Wert von 5,1 und einer Temperatur von 60 bis 65°C ebenfalls dem obersten Boden zugeführt.

Am oberen Ende der Extraktionskolonne fallen stündlich 51 111 kg/h einer Benzol-Lactam-Lösung folgender Zusammensetzung, 0,9% Lactam, 0,2% $H_2O$, 98,9% Benzol, an. Diese Lösung wird zur extraktiven Reinigung des Rohlactam eingesetzt und das Benzol nach destillativer Trennung, wieder zur Extraktion der beschriebenen wäßrigen Phase verwendet.

Am unteren Ende der Kolonne fallen stündlich 20 t einer braunen, wäßrigen Phase an, die Ammonsulfat, Verunreinigungen, 0,2% Caprolactam und 0,4% Oligomere enthält. Der pH-Wert beträgt 4,6. Diese Lösung wird durch Strippen mit Dampf vom Benzol befreit, welches in den Kreislauf zurückgeführt wird. Diese vom Benzol befreite Lösung kann der biologischen Behandlung zugeführt oder nach Aufkonzentration verbrannt werden. Auch nach 4monatigem Betrieb treten keine Verstopfungsprobleme der Siebböden auf.

## Patentansprüche

1. Verfahren zur kontinuierlichen Extraktion von Caprolactam aus solches und dessen Oligomere enthaltenden wäßrigen Lösungen mit organischen Lösungsmitteln, wobei man im oberen Teil einer Extraktionszone eine Caprolactam und dessen Oliogomere enthaltende wäßrige Lösung und im unteren Teil organische Lösungsmittel zuführt, am oberen Ende eine Lösung von Caprolactam in organischen Lösungsmitteln und am unteren Ende eine Oligomere des Caprolactams enthaltende wäßrige Phase abführt, dadurch gekennzeichnet, daß man flüssige aromatische Kohlenwasserstoffe verwendet und die Extraktion bei einem pH-Wert von 2,0 bis 6,0 durchführt.

2. Verfahren nach Anspruch 1, dadurch gekennzeichnet, daß man Benzol oder Toluol als Lösungsmittel verwendet.

3. Verfahren nach den Ansprüchen 1 und 2, dadurch gekennzeichnet, daß man einen pH-Wert von 2,5 bis 5,0 einhält.

4. Verfahren nach den Ansprüchen 1 bis 3, dadurch gekennzeichnet, daß man je Gewichtsteil wäßrige Lösung, die 2- bis 12fache Gewichtsmenge an Benzol oder Toluol verwendet.

## Claims

1. A process for continuously extracting caprolactam from an aqueous solution, containing caprolactam and its oligomers, with organic solvents, by introducing the aqueous solution into the upper part' of an extraction zone and organic solvents into the lower part, and taking off a solution of caprolactam in organic solvents at the upper end and an aqueous phase containing caprolactam oligomers at the lower end, wherein liquid aromatic hydrocarbons are used and the extraction is carried out at a pH of from 2.0 to 6.0.

2. A process as claimed in claim 1, wherein benzene or toluene is used as the solvent.

3. A process as claimed in claims 1 and 2, wherein the pH is kept at from 2.5 to 5.0.

4. A process as claimed in claims 1 to 3, wherein from 2 to 12 parts by weight of benzene or toluene are used per part by weight of aqueous solution.

## Revendications

1. Procédé pour l'extraction continue de la caprolactame de solutions aqueuses contenant celle-ci et ses oligomères à l'aide de solvants organiques, dans lequel on introduit dans la partie supérieure d'une zone d'extraction une solution aqueuse contenant de la caprolactame et des oligomères de celle-ci et dans la partie inférieure des solvants organiques et on soutire au sommet une solution de caprolactame dans les solvants organiques et à l'extrémité inférieure une phase aqueuse contenant les oligomères de la caprolactame, caractérisé en ce que l'on emploie des hydrocarbures aromatiques liquides et on procède à l'extraction à un pH de 2,0 à 6,0.

2. Procédé suivant la revendication 1, caractérisé en ce que l'on emploie comme solvant du benzène ou du toluène.

3. Procédé suivant l'une des revendications 1 et 2, caractérisé en ce que l'on règle le pH entre 2,5 et 5,0.

4. Procédé suivant l'une des revendications 1 à 3, caractérisé en ce que, pour chaque partie en poids de la solution aqueuse, on emploie deux à douze parties en poids de benzène ou de toluène.